Europäisches Patentamt

European Patent Office

Office européen des brevets

(19)

(11) Veröffentlichungsnummer: **0 008 459**
**B1**

(12) # EUROPÄISCHE PATENTSCHRIFT

(45) Veröffentlichungstag der Patentschrift:
21.04.82

(51) Int. Cl.³: **C 07 C 45/50,** C 07 C 47/225

(21) Anmeldenummer: **79103081.0**

(22) Anmeldetag: **22.08.79**

(54) **Verfahren zur Herstellung von 3-(4-Methyl-3-cyclohexen-1-yl)butyraldehyd.**

(30) Priorität: **28.08.78 DE 2837480**

(43) Veröffentlichungstag der Anmeldung:
**05.03.80 Patentblatt 80/5**

(45) Bekanntmachung des Hinweises auf die Patenterteilung.
**21.04.82 Patentblatt 82/16**

(84) Benannte Vertragsstaaten:
**DE FR GB NL SE**

(56) Entgegenhaltungen:
**DE-A-1 939 322**
**DE-A-2 654 799**
**DE-A-2 735 639**
**DE-A-2 753 644**
**US-A-3 499 932**
**US-A-3 499 933**

(73) Patentinhaber: **Ruhrchemie Aktiengesellschaft,
Bruchstrasse 219, D-4200 Oberhausen 13 (DE)**

(72) Erfinder: **Bahrmann, Helmut, Dr. Dipl.-Chem., Im
Freihof 56, 4224 Hünxe (DE)**
Erfinder: **Cornils, Boy, Dr. Dipl.-Chem.,
Friedrich-Ebert-Strasse 45, 5220 Dinslaken (DE)**
Erfinder: **Frohning, Carl Dieter, Dr. Dipl.-Chem,
Elsenbruch 1, 4200 Oberhausen 13 (DE)**
Erfinder: **Weber, Jürgen, Dr. Dipl.-Chem.,
Bunsenstrasse 17, 4200 Oberhausen 13 (DE)**

Verfahren zur Herstellung von 3-(4-Methyl-3-cyclohexen-1-yl)butyraldehyd

Die vorliegende Erfindung betrifft ein Verfahren zur Herstellung von 3-(4-Methyl-3-cyclohexen-1-yl)butyraldehyd durch Umsetzung von Limonen mit Wasserstoff und Kohlenmonoxid in Gegenwart von komplexen Rhodiumverbindungen als Katalysator.

3-(4-Methyl-3-cyclohexen-1-yl)butyraldehyd findet als Bestandteil von Parfüm-Kompositionen Anwendung. Zu seiner Herstellung geht man von Limonen aus, das in Gegenwart von Cobaltcarbonylverbindungen, die neben Kohlenmonoxid zusätzlich Trialkyl- bzw. Triarylphosphine als Liganden enthalten oder in Gegenwart von Rhodiumcarbonylkomplexen mit Kohlenmonoxid und Wasserstoff umgesetzt wird (vgl. C. A., Vol. 79 [1973], 92 400 n).

Die Selektivität dieses Verfahrens ist unbefriedigend, da der gewünschte Aldehyd nur in einer Ausbeute von 42 – 52%, bezogen auf eingesetztes Limonen gebildet wird. Aus diesem Grund ist es für eine industrielle Nutzung nicht geeignet.

Auch in der DE-A-1 939 322 wird die Hydroformylierung von Limonen erwähnt. Abgesehen davon, daß die gewählte Temperatur von 25°C zu Reaktionsgeschwindigkeiten führt, die einer wirtschaftlichen Durchführung des Verfahrens entgegenstehen, ist auch seine Selektivität unbefriedigend.

Aus der US-A-3 499 932 ist die Dihydroformylierung von Dicyclopentadienen mit Kohlenoxid und Wasserstoff in Gegenwart einer katalytischen Menge eines Rhodium-CO-Triphenylphosphin-Komplexes und überschüssigem Triphenylphosphin bei 60 – 140°C und 10 – 200 atm zu Dialdehyden bekannt. Es konnte nicht erwartet werden, daß diese Arbeitsweise zur Dihydroformylierung von cyclischen Doppelbindungen bei der Übertragung auf die Hydroformylierung von Limonen so wie sie in der vorstehend erwähnten Veröffentlichung C. A., Vol. 79 (1973), 92 400 n, beschrieben ist, zu einer selektiven Anlagerung der Formylgruppe ausschließlich an dem endständigen C-Atom der außerhalb des Ringes befindlichen Doppelbindung führt.

Es bestand daher die Aufgabe, einen Prozeß zu entwickeln, der es erlaubt, Limonen sehr selektiv und mit hoher Ausbeute in 3-(4-Methyl-3-cyclohexen-1-yl)butyraldehyd zu überführen.

Gegenstand der Erfindung ist dementsprechend ein Verfahren zur Herstellung von 3-(4-Methyl-3-cyclohexen-1-yl)butyraldehyd durch Hydroformylierung von Limonen in Gegenwart von Katalysatoren, die Metalle der VIII. Gruppe des Periodensystems enthalten, wobei als Katalysatoren Rhodiumcarbonylkomplexe, die zusätzlich Triphenyl- oder Trialkylphosphine als Liganden enthalten eingesetzt, und Drücke von 100 bis 350 bar sowie Temperaturen von 110 bis 160°C angewendet werden und die Triphenyl- bzw. Trialkylphosphine in einer Menge von 10 bis 50 Mol/g Atom Rhodium im Reaktionsgemisch vorhanden sind.

Durch Anwendung der ausgewählten Reaktionsbedingungen können bis zu 98% des Ausgangsstoffes in den gewünschten Aldehyd übergeführt werden.

Der Einsatz der erfindungsgemäßen Katalysatoren stellt sicher, daß die Hydroformylierung des Limonens selektiv in der Weise erfolgt, daß die Formylgruppe ausschließlich an das endständige Kohlenstoffatom der außerhalb des Ringes befindlichen Doppelbindung wandert. Der Rhodiumkatalysator ist bereits in geringer Konzentration wirksam. Üblicherweise setzt man 50 bis 200 ppm Rhodium als Metall, bezogen auf das Olefin, ein.

Grundsätzlich ist es möglich, Rhodium auch in größerer Konzentration zu verwenden; man erzielt dadurch eine Erhöhung der Reaktionsgeschwindigkeit. Dem Einsatz großer Katalysatormengen steht der hohe Rhodiumpreis entgegen, der die Wirtschaftlichkeit des Verfahrens maßgeblich beeinflußt.

Durch Einhalten eines Druckes von 100 bis 350 bar erreicht man darüber hinaus, daß die Umsetzung mit hoher Reaktionsgeschwindigkeit abläuft.

Bedeutsam für das erfindungsgemäße Verfahren ist ferner, daß das Alkylphosphin bzw. Arylphosphin in großem Überschuß, bezogen auf das vorhandene Rhodium, eingesetzt wird. Durch Anwendung von 10 bis 50 Mol Phosphin/g Atom Rhodium wird sichergestellt, daß der Rhodiumkomplex während der Reaktion stets Phosphinliganden enthält.

Grundsätzlich ist es zwar möglich, die Umsetzung ohne Lösungsmittel durchzuführen, die Gegenwart eines Lösungsmittels hat sich jedoch bewährt. Geeignete Lösungsmittel sind insbesondere aliphatische und aromatische Kohlenwasserstoffe, z. B. Hexan, Octan, Benzol, Toluol, Xylol. Üblicherweise setzt man das Lösungsmittel in der gleichen Gewichtsmenge wie das Olefin ein, doch ist es auch möglich, das Lösungsmittel im Überschuß, z. B. im Gewichtsverhältnis 2 : 1, zu verwenden. Der Zusatz eines Lösungsmittels zum Reaktionsgemisch erhöht die Selektivität der Umsetzung hinsichtlich der Bildung des 3-(4-Methyl-3-cyclohexen-1-yl)butyraldehyds. Eine Beschränkung der Verwendung von Lösungsmitteln kann sich aber wiederum aus wirtschaftlichen Überlegungen ergeben.

Die Reaktion kann kontinuierlich oder diskontinuierlich durchgeführt werden. Olefin, Lösungsmittel und Katalysator werden in einem geeigneten Reaktor vorgelegt und nach Erreichen der Reaktionstemperatur Kohlenmonoxid und Wasserstoff zugeleitet, wobei der Gasstrom so bemessen wird, daß sich der gewählte Druck einstellt. Der Wasserstoffanteil im Kohlenmonoxid/Wasserstoff-Gemisch beträgt üblicherweise etwa 30 bis 70 Vol.-%. Der Katalysator kann dem Reaktionsgemisch vorgebildet zugesetzt werden.

Besonders bewährt hat es sich, den Katalysator in situ aus einem Rhodiumsalz, z. B. Rhodium-2-ethylhexanoat, dem Phosphin sowie Kohlenmonoxid und Wasserstoff zu formieren. In diesem Fall legt man Lösungsmittel, Rhodiumsalz und Phosphin vor, führt Kohlenmonoxid und Wasserstoff zu und leitet nach Bildung des katalytisch wirksamen Komplexes das Olefin ein.

Nach Beendigung der Umsetzung wird der gebildete Aldehyd destillativ vom Reaktionsprodukt abgetrennt. Im Destillationsrückstand verbleibt der Katalysator, der zusammen mit dem Lösungsmittel in die Reaktion zurückgeführt werden kann. Es ist aber auch möglich, zunächst den Katalysator in bekannter Weise, z. B. durch Behandlung des Reaktionsgemisches mit Wasserdampf oder Wasserstoff abzutrennen und dann die Aufarbeitung des Reaktionsproduktes vorzunehmen.

Zur weiteren Reinigung des 3-(4-Methyl-3-cyclohexen-1-yl)butyraldehydes kann nochmals fraktioniert destilliert werden.

## Patentansprüche

1. Verfahren zur Herstellung von 3-(4-Methyl-3-cyclohexen-1-yl)butyraldehyd durch Hydroformylierung von Limonen in Gegenwart von Katalysatoren, die Metalle der VIII. Gruppe des Periodensystems enthalten, dadurch gekennzeichnet, daß als Katalysatoren Rhodiumcarbonylkomplexe, die zusätzlich Triphenyl- oder Trialkylphosphine als Liganden enthalten, eingesetzt und Drücke von 100 bis 350 bar sowie Temperaturen von 110 bis 160°C angewendet werden und die Triphenyl- bzw. Trialkylphosphine in einer Menge von 10 bis 50 Mol/g-Atom Rhodium im Reaktionsgemisch vorhanden sind.

2. Verfahren nach Anspruch 1, dadurch gekennzeichnet, daß die Umsetzung in Gegenwart eines Lösungsmittels, insbesondere eines aliphatischen oder aromatischen Kohlenwasserstoffes, vorgenommen wird.

## Claims

1. A process for the preparation of 3-(4-methyl-3-cyclohexen-1-yl)butyraldehyde by hydroformylation of limonene in the presence of catalysts containing metals of Group VIII of the Periodic Table, characterized in that rhodium carbonyl complexes containing additionally triphenyl or trialkyl phosphines as ligands are used as catalysts and pressures of 100 to 350 bars and temperatures of 110 to 160°C are used and the triphenyl or trialkyl phosphines are present in an amount of 10 to 50 mmoles per gram atom of rhodium in the reaction mixture.

2. A process according to claim 1, characterized in that the reaction is carried out in the presence of a solvent, especially an aliphatic or aromatic hydrocarbon.

## Revendications

1. Procédé de préparation du 3-(4-méthyl-3-cyclohexène-1-yl)-butyraldéhyde par hydroformylation du limonène en présence de catalyseurs contenant des métaux du groupe VIII de la Classification Périodique, caractérisé en ce que l'on utilise comme catalyseurs des complexes carbonylés du rhodium contenant en outre des triphényl- ou trialkyl-phosphines en tant que ligands, on opère à des pressions de 100 à 350 bars et à températures de 110 à 160°C et les triphényl- ou trialkyl-phosphines sont présentes en quantités de 10 à 50 moles par atome-gramme de rhodium dans le mélange de réaction.

2. Procédé selon la revendication 1, caractérisé en ce que l'on effectue la réaction en présence d'un solvant, en particulier un hydrocarbure aliphatique ou aromatique.